# EUROPÄISCHE PATENTSCHRIFT

(11) **EP 2 777 761 B1**
(45) Veröffentlichungstag und Bekanntmachung des Hinweises auf die Patenterteilung: **29.03.2017**
(21) Anmeldenummer: 14153598.9
(22) Anmeldetag: 03.02.2014
(51) Int. Cl.: A61N 1/05

(54) **Implantierbare Elektrode**
Implantable electrode
Électrode implantable

(30) Priorität: 14.03.2013 US 201361781110 P
(43) Veröffentlichungstag der Anmeldung: 17.09.2014
(73) Patentinhaber: BIOTRONIK SE & Co. KG, 12359 Berlin (DE)
(72) Erfinder: Kolberg, Gernot, 12161 Berlin (DE)
(74) Vertreter: Galander, Marcus

(56) Entgegenhaltungen:
- EP-A2- 1 832 309
- WO-A2-2013/049601
- US-A1- 2007 135 881
- US-B2- 6 985 774
- US-B2- 7 643 886
- US-B2- 8 012 149

## Beschreibung

Die Erfindung betrifft eine implantierbare Elektrode umfassend einen langgestreckten Elektrodenkörper und mindestens ein Lumen, das sich über zumindest eine Teillänge des Elektrodenkörpers darin erstreckt.

Grundsätzlich sind solche Elektroden zur Implantation im oder am Herzen, in Gefäßen, in oder auf Muskeln, im Gehirn, Rückenmark, entlang von Nervensträngen und dergleichen vorgesehen. Je nach Anwendungszweck können sie mit Elektrodenflächen zur elektrophysiologischen Stimulation und/oder zur entsprechenden Wahrnehmung von elektrophysiologischen Körpersignalen und/oder mit Sensoren zur Aufnahme von jedweden physiologisch relevanten Informationen und/oder mit Aktuatoren zur Manipulation im Körper oder zur Ausschüttung von Medikamenten versehen sein.

Die der Erfindung zugrunde liegende Problematik soll nun am Beispiel einer Herzelektrode erläutert werden. Damit diese einen optimalen Effekt gemäß ihres therapeutischen Zwecks erzielen kann, ist eine exakte Platzierung der Elektrode im Herzen wichtig. Insbesondere bei einem transluminaren Vorschieben muss die Elektrode eine gewisse axiale Stabilität aufweisen. Ferner kann es für bestimmte Zielorte im Körper hilfreich sein, dass die Elektrode variabel einen Bogen ausprägen kann oder diesen schon in der Form vorgegeben zeigt.

Die mit diesen mechanischen Eigenschaften verbundene Steifigkeit ist insbesondere bei einer permanent implantierten Elektrode problematisch. In der Langzeitanwendung sollen solche Elektroden nämlich möglichst weich sein, um das Herz so wenig wie möglich mechanisch zu belasten. Auch zeigt eine flexible Elektrode in der Regel eine erhöhte Dauerstabilität.

Aus dem Stand der Technik sind zur Lösung der vorstehend erörterten Problematik verschiedene Techniken bekannt. So kann die Elektrode beim Implantieren durch einen Draht - ein sogenanntes Stilett oder ein Mandrin - versteift werden. Dazu weist die Elektrode ein inneres Lumen auf, das für die Prozedur der Implantation den Draht aufnehmen kann. Damit wird die Elektrode versteift und kann in den Gefäßen vorgeschoben werden. Der Draht kann ferner vorgebogen sein, wodurch er der Elektrode bei Bedarf Vorformen aufprägt. Da ein Draht schnell aus dem Elektrodenkörper gezogen werden kann, kann eine Verformung sehr einfach variiert und den entsprechenden Bedürfnissen angepasst werden. Es sind auch Ausführungen aus dem Stand der Technik bekannt, bei der die Biegung des Drahtes während der Anwendung variiert werden kann.

Ein weiterer vorbekannter Lösungsansatz liegt in der Verwendung vorgeformter Elektroden, beispielsweise sogenannter J-Elektroden. Diese können besonders leicht im Vorhof des Herzens verankert werden, da sie im distalen Bereich eine aufgeprägte Kurve zeigen, die die Elektrode nach dem Ziehen eines Versteifungsdrahtes in die gewünschte Position biegt.

Weiterhin ist es bekannt, die Elektrode durch einen Katheter zu führen. Die Einführbestecke sind in diesem Falle herkömmlicher Weise kurz und dienen nur dazu, die Elektrode in das Gefäßsystem zu schieben und am Einlass in ein Gefäß zu führen. Insbesondere bei Anwendungen im linken Herzteil werden Katheter mit speziellen Kurvenformen verwendet, die leicht an gewünschte Orte geführt werden können. Durch diese Katheter kann dann die Elektrode vorgeschoben werden. Es sind in diesem Zusammenhang sehr dünne Elektroden bekannt, die auf ein Führungsdrahtlumen verzichten. Für die Implantation wird dann ein spezieller Katheter benötigt.

Eine Elektrode kann auch auf einem Draht geführt werden. Es kommt dabei ein weicher Draht zum Einsatz, der mit Hilfe eines Katheters als Platzhalter an eine gewünschte Position geschoben wird. Über diesen Draht wird dann eine offene Elektrode an die entsprechende Stelle im Körper vorgeschoben.

Die vorgenannten Elektrodentypen, die ein Stilett, einen Mandrin oder einen Führungsdraht verwenden, weisen für diese Hilfskomponenten ein radial stabilisiertes Lumen auf, mit dem die Elektrode leichtgängig über diese Komponenten gleiten kann. Das Lumen wird dabei in der Regel durch eine Wendel realisiert, die zusätzlich die Funktion einer Zuleitung und bei aktiv fixierbaren Elektroden einer Torsionsübertragung übernimmt. Dieses Lumen kann nach der Implantation nicht eliminiert werden, da die Wendel fest in dem Elektrodenkörper integriert ist. Insoweit kann die Elektrode nach einer Implantation nicht flexibler ausgelegt werden.

Ferner ist die Steifheit von Führungsdrähten von ihrem Durchmesser abhängig. Aufgrund eines entsprechenden Mindestdurchmessers solcher Drähte kann auch das dafür vorgesehene Führungslumen nicht verkleinert werden. Das Führungslumen erweist sich so für die gewünschte Durchmesserreduktion von Elektroden als limitierende Größe.

Elektroden, die bauartbedingt kein Führungslumen besitzen, benötigen einen aufwändigen und umständlich zu bedienenden Führungskatheter.

Weitere, aus dem druckschriftlichen Stand der Technik bekannte Vorrichtungen sehen zumeist flüssigkeitsbetätigte Veränderungen, insbesondere des Durchmessers von Abschnitten einer implantierbaren Elektrode vor. Diese mit dem weiter oben beschriebenen mindestens einen Lumen verbundenen Abschnitte dienen in der Regel dazu, reversibel Wandkontakt herzustellen oder die implantierbare Elektrode durch eine Umfangserweiterung in einem Gefäß zu fixieren. Dazu sehen die implantierbaren Elektroden eine Druckeinrichtung vor, mittels derer das mindestens eine Lumen mit einem Druckmedium derart reversibel beaufschlagbar ist, dass der Elektrodenkörper abhängig vom Druck des Druckmediums seine Flexibilität und/oder Form variiert.
Beispiele solcher Elektrodenleitungen finden sich in den Dokumenten EP 1 832 309 A2, US 2007/0135881 A1, US 7,643,886 B2 oder US 8,012,149 B2.

Ausgehend von den geschilderten Problemen des Standes der Technik liegt der Erfindung die Aufgabe zugrunde, eine implantierbare Elektrode bei guter, zweckangepasster Steuerbarkeit im Durchmesser geringer und im Dauereinsatz flexibler zu gestalten. Insbesondere soll die Elektrode für die Zeit der Implantation temporär stabilisiert und gegebenenfalls vorgeformt werden, ohne ein dauerhaft offenes Lumen für einen Führungsdraht vorsehen zu müssen oder einen Katheter zu benötigen.

Diese Aufgabe wird konzeptionell durch die im Kennzeichnungsteil des Anspruches 1 angegebenen Merkmale gelöst, wonach der Elektrodenkörper zumindest abschnittsweise eine Vorformung in einer Helix-Form aufweist, die durch die Druckbeaufschlagung des mindestens einen Lumen temporär aufhebbar ist.

Vorteilhafterweise können auf der Basis dieser erfindungsgemäßen Lösung dünnere Elektroden konzipiert werden, da das mit dem Druckmedium zu beaufschlagende Lumen im Querschnitt deutlich kleiner ausgeführt werden kann, als ein Lumen für einen Führungsdraht. Durch die entsprechende Auslegung der Lumen und der sie umgebenden Materialbereiche des Elektrodenkörpers kann - wie die in den abhängigen Ansprüchen angegebenen vorteilhaften Weiterbildungen zeigen - das Funktionsspektrum solcher Elektroden deutlich erweitert werden, da deren Eigenschaften für die Zeit der Implantationsprozedur deutlich flexibler zu verändern sind als beim Stand der Technik. Ferner lassen sich neue Implantationshilfen in die Produkte einbauen, die die Implantationsprozedur erheblich vereinfachen können.

Im Einzelnen ist bevorzugtermaßen vorgesehen, das mindestens eine Lumen mit einem flüssigen Druckmedium, vorzugsweise mit Natriumchlorid-Lösung oder einem geeigneten Gel, zu füllen. Das Drucksystem ist also hydraulisch ausgelegt.

In einer bevorzugten Ausführungsform kann das Lumen mit einem entsprechenden kompressiblen, vorzugsweise manuell betätigbaren Reservoir für das Druckmedium ein geschlossenes Hydrauliksystem bilden. Dieses Reservoir kann beispielsweise im Bereich des proximalen Steckers der Elektrode platziert sein und durch Kompression beispielsweise durch Druck mit den Fingern oder dauerhaft durch Anlegen einer Ligatur den Druck im Gesamtsystem erhöhen. So kann die Elektrode während der Implantation jederzeit vom Operateur ohne Werkzeug durch Druckerhöhung versteift oder durch Druckerniedrigung entspannt werden.

Gemäß einer weiteren alternativen Ausführungsform kann eine Einspritzvorrichtung für das Druckmedium lösbar mit dem mindestens einen Lumen der Elektrode verbunden werden. In der Praxis kann hierzu der Mandrinzugang eines herkömmlichen Steckers für den Druckanschluss genutzt werden. Über einen sogenannten Luerlock-Adapter wird dann Kochsalzlösung über eine angeschlossene Spritze eingespritzt und mit definiertem Druck beaufschlagt.

Die Variierung des Drucks durch die vorstehenden Maßnahmen kann zu verschiedenen Eigenschaftsänderungen des Elektrodenkörpers genutzt werden. So kann das mindestens eine Lumen in definierten Versteifungsbereichen durch den Druck des Druckmediums zu einem dort druckabhängig versteiften Elektrodenkörper führen. Aus Sicherheitsgründen kann in diesen Versteifungsbereichen dann das Material, das das Lumen umgibt, eine Streckgrenze aufweisen, die vorzugsweise noch durch eine Faser- oder Füllstoffverstärkung des Materials einstellbar ist.

Eine alternative oder zusätzliche bevorzugte Weiterbildung sieht vor, dass das mindestens eine Lumen in definierten Ausdehnungsbereichen durch den Druck des Druckmediums zu einem dort im Durchmesser druckabhängig formveränderten, insbesondere vergrößerten oder gekrümmten Elektrodenkörper führt. Bevorzugterweise kann diese Durchmesser- oder Formänderung durch ein in Falten gelegtes Lumen in den definierten Ausdehnungsbereichen realisiert werden, wobei die Falten durch den Druck des Druckmediums sich entfalten können.

Allen Form- und Flexibilitätsänderungen ist gemeinsam, dass diese durch Ablassen des Drucks reversibel sind, so dass nach der erfolgten Implantationsprozedur die Elektrode einen minimalen Durchmesser und maximale Flexibilität aufweist.

Bevorzugte Materialien für eine Auskleidung oder Umhüllung des Lumens sind dünnwandige, reißfeste Materialien mit einer Streckgrenze, wie z. B. PET oder PE, die besonders gut vor einem Aufreißen der Lumenwand schützen. Dadurch ist eine gute Versteifung des Elektrodenkörpers möglich, wobei die Lumen bei einer Druckbeaufschlagung den Durchmesser des Elektrodenkörpers erhöhen, sofern der endgültige Durchmesser noch nicht erreicht ist. Sofern das Lumen mit einer dünnwandigen, reißfesten Materialauskleidung versehen ist, aber bereits ohne Druckbeaufschlagung den endgültigen Durchmesser aufweist, versteift sich bei einer Druckbeaufschlagung das Lumen, ohne den Durchmesser des Elektrodenkörpers zu erhöhen.

Die oben erwähnte Streckgrenze des Materials kann in bevorzugter Weise in den Versteifungsbereichen des Lumens durch eine Faser- oder Füllstoffverstärkung des das Lumen umgebenden Materials eingestellt werden.

Eine zusätzliche oder alternative Weiterbildung für den Elektrodenkörper sieht vor, dass das mindestens eine Lumen in definierten Ausdehnungsbereichen durch den Druck des Druckmediums zu einem dort im Durchmesser druckabhängig formveränderten, insbesondere vergrößerten oder gekrümmten Elektrodenkörper führt. Dazu ist es von Vorteil, dass sich das Lumen in diesen Ausdehnungsbereichen im dehnbaren Material des Elektrodenkörpers befindet. Solche Materialien können beispielsweise Silikone, Polyurethane, Copolymere und so weiter sein. Der Verformungseffekt lässt sich dann durch eine Variation des Druckes steuern.

Bevorzugte Konfigurationen für die Lumenauslegung sehen die Bildung eines sternförmigen Querschnitts des Lumens durch die Falten vor, wobei der Querschnitt durch Beaufschlagung durch das Druckmedium in einem kreisförmig expandierten Querschnitt übergehen kann.

Eine weitere Alternative für eine Verformung des Elektrodenkörpers besteht darin, den Elektrodenkörper über eine Soll-Krümmungslänge mit mindestens einem exzentrisch angeordneten, im Querschnitt sichelförmigen Lumen zu versehen. Wird ein solches Lumen unter Druck gesetzt, so krümmt es sich in die zur Exzentrität des Lumens entgegengesetzten Richtung, so dass eine durch die Druckbeaufschlagung steuerbare oder in Krümmungsradien legbare Elektrode erzielt wird.

Grundsätzlich kann über die Art der Anordnung und Zahl der Lumen eine große Bandbreite an Form- und Eigenschaftsänderungen entlang des Elektrodenkörpers erzielt werden. So können beispielsweise zwei oder mehr verschiedene, vorzugsweise miteinander über Druckventile verbundene Lumen oder Lumenabschnitte im Elektrodenkörper vorgesehen sein. Insbesondere durch die Druckventile sind diese einzelnen Lumen oder Lumenabschnitte dann über unterschiedliche Druckniveaus des Druckmediums selektiv ansteuerbar, indem die Druckventile beispielsweise bei unterschiedlichen Druckwerten öffnen. So kann die Elektrode dann bei Vergrößerung des Druckes und bei Öffnung eines dann angesteuerten Lumenabschnitts seine Form gezielt verändern.

Wenn sich die zwei oder mehr Lumen in unterschiedlich dehnbaren Materialbereichen des Elektrodenkörpers befinden, so kann die Elektrode bei Druckvergrößerung immer neue Formen annehmen.

Gemäß einer weiteren bevorzugten Ausführungsform der Erfindung können durch die Beaufschlagung des mindestens einen Lumens durch das Druckmedium in Teilbereichen des Elektrodenkörpers variable Funktionsteile, wie Häkchen, Schrauben oder Spiralen zur Verankerung des Elektrodenkörpers im umgebenden Körpergewebe ausgebildet werden. Die Formänderung des Elektrodenkörpers ist so bei der Positionierung und Fixierung der Elektrode hilfreich. Als Beispiel können sich sogenannte Tines temporär versteifen lassen, um die Elektrode tiefer ins Trabekelwerk des Herzgewebes vorzuschieben. In einer anderen Variante können bei Druckschwankungen kleine Häkchen vor- und zurückklappen, die eine sogenannte CS-Elektrode im Gefäß vorwärts wandern lassen. Denkbar ist auch eine durch Druckbeaufschlagung auslösbare Schraubfixierung.

In umgekehrter Weise kann der Elektrodenkörper in einer letzten bevorzugten Ausführungsform zumindest abschnittsweise eine Vorformung, wie beispielsweise eine Helixform, aufweisen, die durch die Druckbeaufschlagung des mindestens einen Lumens im Elektrodenkörper temporär aufhebbar ist. Es kann also für die Implantation beispielsweise im Gefäßsystem durch die Druckbeaufschlagung eine temporäre Begradigung des Elektrodenkörpers realisiert werden, die sich nach Druckentlastung wieder aufhebt, wodurch die Vorform wieder zu Tage tritt.

Grundsätzlich ist darauf hinzuweisen, dass eine erfindungsgemäße Elektrode elektronische Komponenten zum Ansteuern und/oder Auslesen von Elektroden, Sensoren oder Aktuatoren und/oder zum Aufbereiten bzw. Übertragen von Informationen oder Energien aufweisen kann. Diese Maßnahmen haben mit dem Kern der Erfindung an sich nichts zu tun und finden sich daher nicht eigens in abhängigen Ansprüchen wieder.

Weitere Merkmale, Einzelheiten und Vorteile der Erfindung ergeben sich aus der nachfolgenden Beschreibung von Ausführungsbeispielen anhand der beigefügten Zeichnungen. Es zeigen:
- Fig. 1: eine Zusammenstellungszeichnung einer Bradykardie-Elektrode mit IS-1-Standardstecker, einem Druckadapter dafür und einem Druckgeber,
- Fig. 2: eine vergrößerte Schnittdarstellung des Elektrodenkörpers an der Position II gemäß Fig. 1 in drucklosem Zustand,
- Fig. 3: eine Darstellung analog Fig. 2 unter Druckbeaufschlagung,
- Fig. 4: eine Zusammenstellungszeichnung einer ICD-Elektrode mit DF-4-Standardstecker sowie einen Druckadapter dafür,
- Fig. 5: eine vergrößerte Schnittdarstellung des Elektrodenkörpers an der Position V gemäß Fig. 4 in drucklosem Zustand,
- Fig. 6: eine Darstellung analog Fig. 5 unter Druckbeaufschlagung,
- Fig. 7: eine Ansicht einer J-Elektrode mit IS-1-Stecker in drucklosem Zustand,
- Fig. 8 und 9: vergrößerte Schnittdarstellungen der J-Elektrode an den Positionen VIII bzw. IX gemäß Fig. 7,
- Fig. 10: eine Darstellung der J-Elektrode analog Fig. 7 unter Beaufschlagung auf einem ersten Druckniveau,
- Fig. 11 und 12: vergrößerte Schnittdarstellungen der J-Elektrode an den Positionen XI bzw. XII gemäß Fig. 10,
- Fig. 13: eine Ansicht der J-Elektrode gemäß Fig. 7 und 10 unter Beaufschlagung auf einem zweiten, gegenüber dem ersten Druckniveau gemäß Fig. 7 höheren Druckniveau, und
- Fig. 14: eine vergrößerte Schnittdarstellung der J-Elektrode an der Position XIV gemäß Fig. 13.

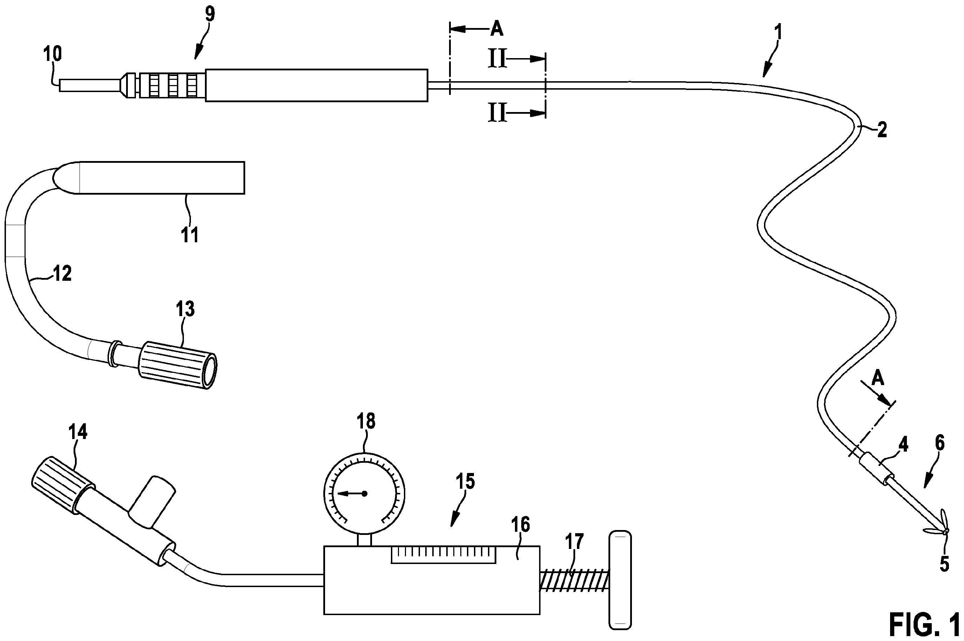

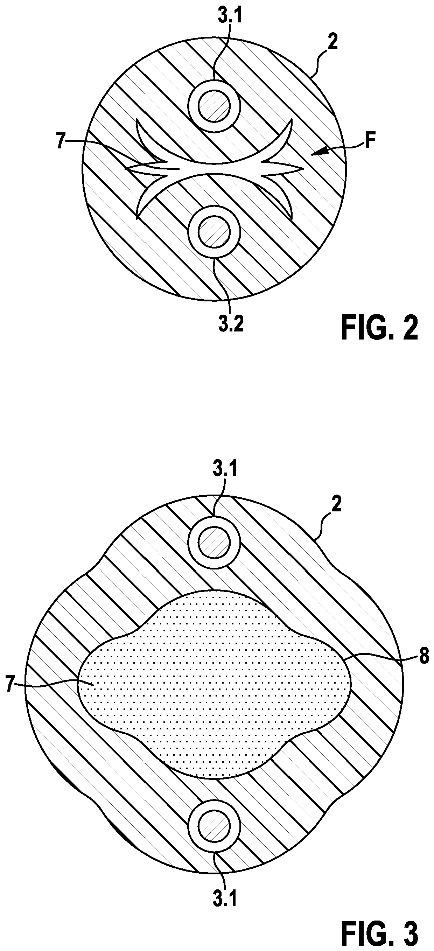

Die in Fig. 1 gezeigte Bradykardie-Elektrode 1 weist einen langgestreckten, sehr dünnen Elektrodenkörper 2 aus einem geeigneten expandierbaren, flexiblen Material, wie beispielsweise Polyurethan, Silicon Polyamid - beispielsweise Nylon, Polyethylen oder Polyethylenterephthalat auf, der - wie Fig. 2 und 3 zeigen - zwei Zuleitungen 3.1, 3.2 für die Ringelektrode 4 und die Spitzenelektrode 5 am distalen Ende 6 des Elektrodenkörpers 2 über nicht eigens herausgezeichnete Lumen im Elektrodenkörper führt. Die beiden Zuleitungen 3.1, 3.2 sind exzentrisch außerhalb der Längsachse des Elektrodenkörpers 2 angeordnet. Koaxial dazu ist ein Lumen 7 in den Elektrodenkörper 2 eingebracht, das mit einer dünnen, reißfesten Wandung 8 versehen ist.

Wie ferner aus Fig. 1 deutlich wird, ist die Bradykardie-Elektrode 1 zum Anschluss der Zuleitungen 3.1, 3.2 mit einem genormten IS-1-Stecker 9 versehen, der zentral einen Ausgang 10 für das Lumen 7 aufweist.

Als Kit ist der Elektrode 1 ein Druckadapter 11 zugeordnet, der auf den Ausgang 10 des IS-1-Steckers 9 druckdicht aufsteckbar ist. Über einen Schlauch 12 ist an den Druckadapter 11 ein Luerlock-Anschluss angebunden, der mit einem Luerlock-Gegenstück an einem PTCA-Druckgeber 15 anschließbar ist. In diesem Druckgeber ist ein Reservoir 16 für ein Druckmedium, wie beispielsweise Kochsalz-Lösung oder ein geeignetes Gel, vorgesehen, das über eine Spindel 17 unter Druck setzbar ist. Der Druck im Reservoir 16 ist über ein Manometer 18 ablesbar.

Wie aus Fig. 2 deutlich wird, ist das Lumen 7 in drucklosem Zustand sternförmig in Falten F zusammengefaltet, so dass der Elektrodenkörper 2 auf seiner gesamten Länge einen minimalen Durchmesser einnimmt und sehr flexibel ist. Der Elektrodenkörper 2 ist damit in einem optimalen Zustand für eine Dauerimplantation im Körper.

Für die Implantationsprozedur kann es von Vorteil sein, dass die Elektrode 1 versteift wird, um einen besseren Vorschub durch die Körpergefäße zu erreichen. Um dies zu realisieren, wird das Lumen 7 über den Druckgeber 15 mit dem Druckmedium gefüllt und damit druckbeaufschlagt. Das Lumen 7 entfaltet sich, der Elektrodenkörper 2 dehnt sich in dem damit gebildeten Ausdehnungsbereich A für den Elektrodenkörper 2 aus und wird erheblich versteift, wie dies in Fig. 3 dargestellt ist. Die Elektrode 1 ist damit temporär für den Implantationsvorgang versteift. Nach dessen Abschluss kann das Druckmedium wieder aus dem Lumen 7 herausgesaugt werden, dies faltet sich zusammen, womit der Elektrodenkörper 2 wieder sehr dünn und flexibel ist.

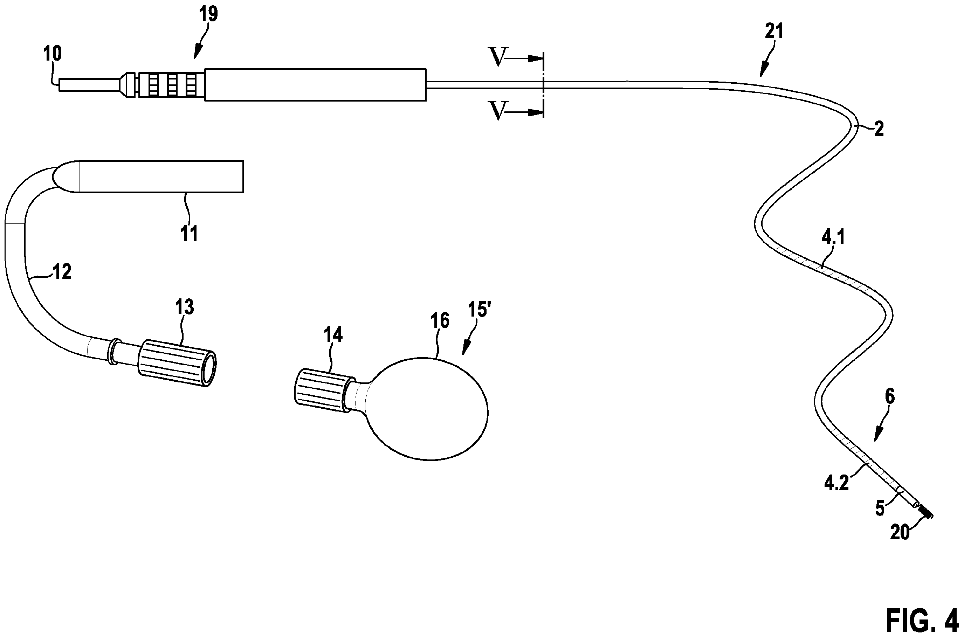

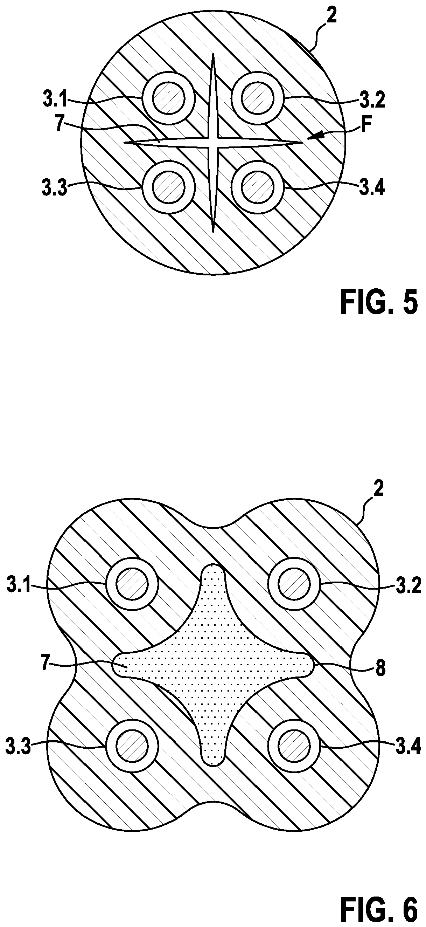

Fig. 4 bis 6 zeigt eine ICD-Elektrode als Ausführungsbeispiel der Erfindung, bei der der Elektrodenkörper 2 statt zweier Lumen mit jeweiliger Zuleitungen 3.1, 3.2 noch zwei weitere Lumen mit Zuleitungen 3.3, 3.4 aufweist, die über einen entsprechenden DF-4-Stecker 19 angeschlossen werden können. Über diese Zuleitungen sind beispielsweise zwei Ringelektrodenflächen 4.1, 4.2 und wiederum eine Spitzenelektrode 5 mit einem Einschraubfortsatz 20 elektrisch anschließbar. Analog dem Ausführungsbeispiel gemäß den Fig. 1 bis 3 ist das zentrale Lumen 7 über einen Druckadapter 11 und ein ballonartiges, geschlossenes Reservoir 15' unter Druck setzbar. Das Lumen 7 ist - wie in den Fig. 5 und 6 gezeigt - aus dem eingefalteten Zustand (Fig. 5 - Falten F) durch die Druckbeaufschlagung über das Ballonreservoir 15' in eine versteifte Konfiguration überzuführen, in der das Lumen 7 expandiert ist (Fig. 6).

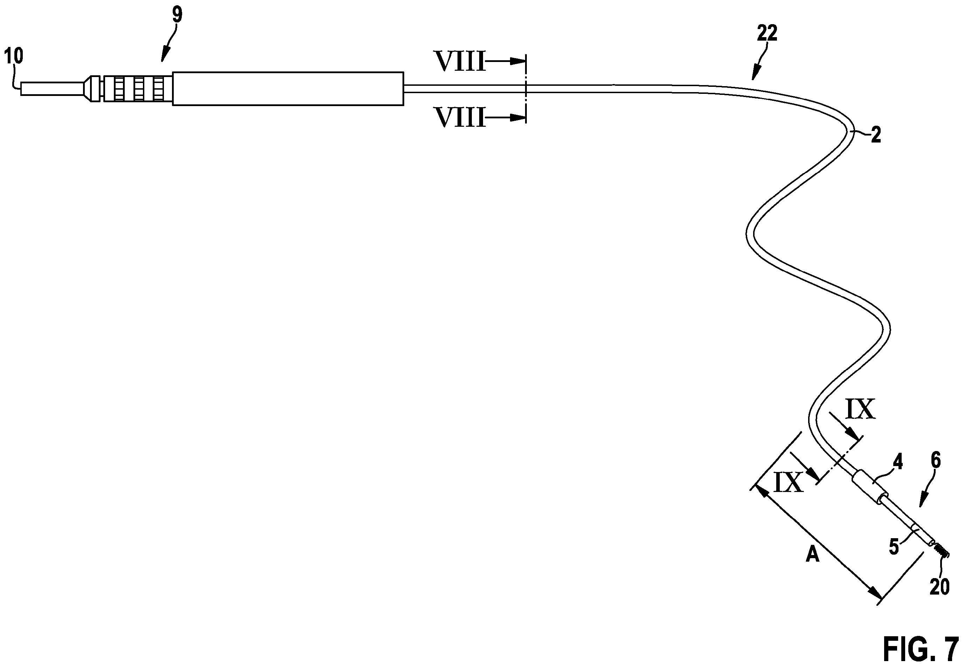

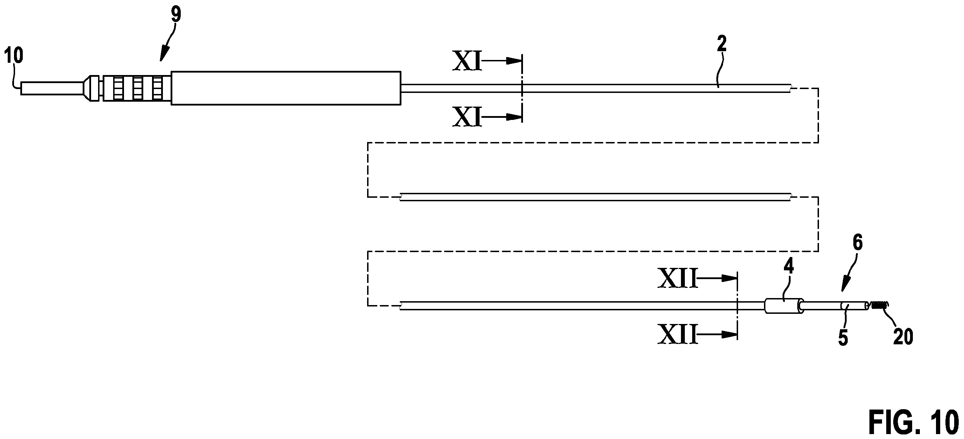

Die Fig. 7 bis 14 zeigen eine weitere Ausführungsform der Erfindung anhand einer sogenannten J-Elektrode, die analog der Ausführungsform gemäß den Fig. 1 bis 3 zwei Zuleitungen 3.1, 3.2 zu einer Ringelektrode 4 und einer Spitzenelektrode 5 aufweist. Der Anschluss dieser J-Elektrode 22 erfolgt wiederum über einen IS-1-Stecker 9, der das Lumen 7 über seinen zentralen Ausgang 10 herausführt.

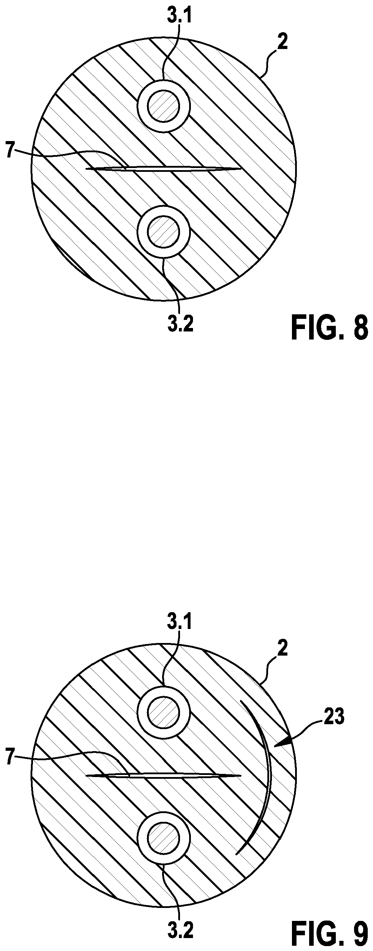

Wie aus Fig. 8 hervorgeht, weist der Elektrodenkörper 2 an einer weit vom distalen Ende 6 entfernten Position entsprechend der Schnittlinie VIII in Fig. 7 ein einziges Lumen 7 auf, das bis zum distalen Ende 6 hindurchgeführt ist, wie dies aus Fig. 9 deutlich wird.

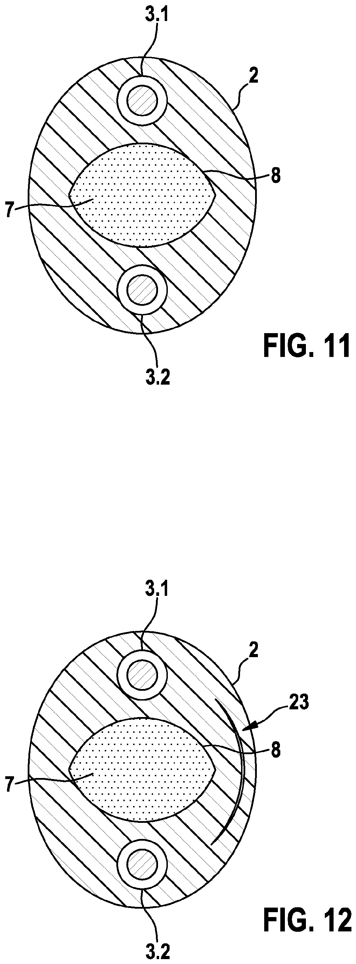

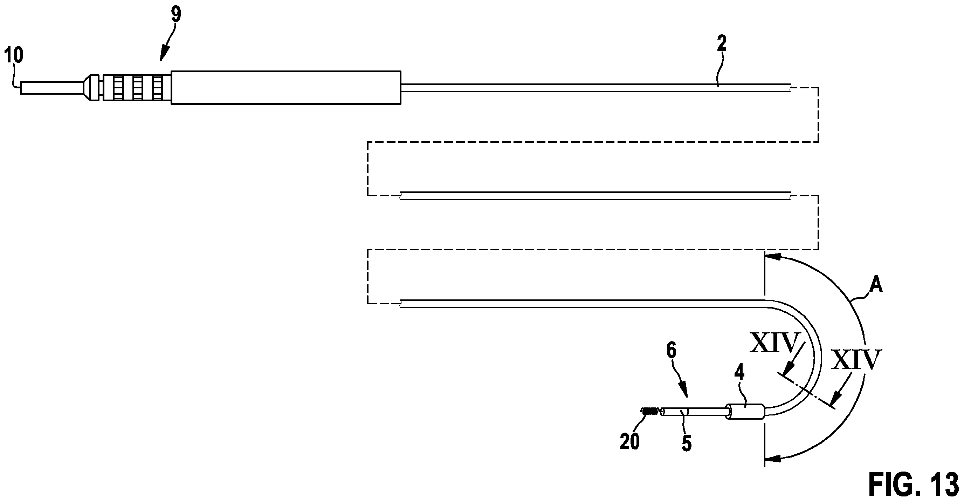

Die J-Elektrode 22 zeichnet sich dadurch aus, dass ihr Elektrodenkörper auf einer Länge von einigen Zentimetern vor dem distalen Ende 6 in eine gekrümmte Bogenform - also eine J-Form - deformiert werden kann, wie dies in Fig. 13 angedeutet ist. Um diese Bogenform reversibel erzeugen zu können, zweigt vom zentralen Lumen 7 vor dem zu erzeugenden Bogen ein zweites Lumen 23 ab, das im Querschnitt sichelförmig ist und exzentrisch mit der Sichelbiegung etwa parallel zur Außenkontur des im Querschnitt kreisförmigen Elektrodenkörpers 2 verläuft, wie dies aus den Fig. 9 und 12 deutlich wird. Dieses zweite Lumen 23 bildet einen Ausdehnungsbereich A für den Elektrodenkörper 2 und endet dann am distalen Ende 6 vor der Ringelektrode 4.

Die beiden Lumen 7 und 23 sind in ihrem Druck-Ansprechverhalten so ausgelegt, dass ausgehend vom drucklosen Zustand zuerst das zentrale Lumen 7 bei Anlegen eines ersten Druckwertes P1 expandiert und sich somit die J-Elektrode 22 über ihre gesamte Länge versteift. Dieser Zustand ist in den Schnittdarstellungen gemäß Fig. 11 und 12 dargestellt.

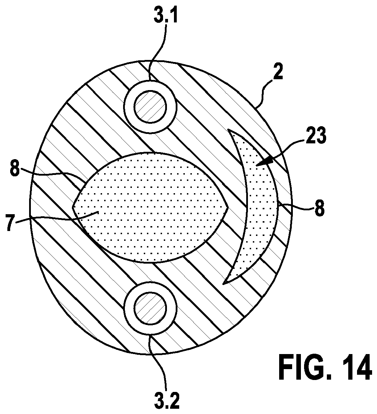

Bei einer weiteren Erhöhung des Druckes auf einen Wert P2 > P1 wird dann das Druckmedium in das abzweigende Lumen 23 eingebracht, so dass dieses aus dem in Fig. 9 und 12 gezeigten, nicht-expandierten Zustand in den in Fig. 14 dargestellten ausgedehnten Zustand aufgepumpt wird. Durch die damit verbundene Volumenausdehnung des auf der Seite des Lumens 23 liegenden Materials des Elektrodenkörpers 2 erfolgt eine Ausdehnung insgesamt, so dass sich der Elektrodenkörper 2 in die in Fig. 13 gezeigte J-Form umbiegt.

Dieses Verhalten ist vergleichbar mit dem Krümmungsmechanismus bei einem Bimetallstreifen. Durch die axial einseitige Ausdehnung des Elektrodenkörpers 2 formt sich dieser in dem 180°-Radius aus, mit dem die Elektrode leicht im Vorhof des Herzens positioniert und dort fixiert werden kann.

Nach dem Absaugen des Druckmediums (z. B. Kochsalzlösung) ist die Elektrode dann wieder sehr dünn und hoch flexibel.

Die unterschiedlichen Ansprechparameter der beiden Lumen 7 und 23 können beispielsweise durch unterschiedlich steife Wandungsmaterialien oder durch ein in den Zeichnungen nicht dargestelltes Druckventil am Abzweig des Lumens 23 vom Lumen 7 eingestellt werden.

## Patentansprüche

1. Implantierbare Elektrode umfassend
- einen lang gestreckten Elektrodenkörper (2),
- mindestens ein Lumen (7, 23), das sich über zumindest eine Teillänge des Elektrodenkörpers (2) darin erstreckt, und
- eine Druckeinrichtung (15), mittels derer das mindestens eine Lumen (7, 23) mit einem Druckmedium derart reversibel beaufschlagbar ist, dass der Elektrodenkörper (2) abhängig vom Druck des Druckmediums seine Flexibilität und/oder Form variiert,
**gekennzeichnet dadurch, dass**
der Elektrodenkörper (2) zumindest abschnittsweise eine Vorformung in einer Helix-Form aufweist, die durch die Druckbeaufschlagung des mindestens einen Lumens temporär aufhebbar ist.

2. Elektrode nach Anspruch 1, **dadurch gekennzeichnet, dass** das mindestens eine Lumen (7, 23) mit einem flüssigen Druckmedium, vorzugsweise mit Natriumchlorid-Lösung oder einem Gel, gefüllt ist.

3. Elektrode nach Anspruch 2, **dadurch gekennzeichnet, dass** das mindestens eine mit dem flüssigen Druckmedium gefüllte Lumen (7, 23) mit einem kompressiblen, vorzugsweise manuell betätigbaren Reservoir (15') ein geschlossenes Hydrauliksystem bildet.

4. Elektrode nach Anspruch 1 oder 2, **dadurch gekennzeichnet, dass** eine Einspritzvorrichtung (15) für das Druckmedium lösbar mit dem mindestens einen Lumen (7, 23) der Elektrode verbindbar ist.

5. Elektrode nach einem der vorgenannten Ansprüche, **dadurch gekennzeichnet, dass** das mindestens eine Lumen (7, 23) in definierten Versteifungsbereichen durch den Druck des Druckmediums zu einem dort druckabhängig versteiften Elektrodenkörper (2) führt.

6. Elektrode nach Anspruch 5, **dadurch gekennzeichnet, dass** in den Versteifungsbereichen das das Lumen (7, 23) umgebende Material eine Streckgrenze aufweist, die vorzugsweise durch eine Faser- oder Füllstoffverstärkung des Materials einstellbar ist.

7. Elektrode nach einem der vorgenannten Ansprüche, **dadurch gekennzeichnet, dass** das mindestens eine Lumen (7, 23) in definierten Ausdehnungsbereichen (A) durch den Druck des Druckmediums zu einem dort im Durchmesser druckabhängig formveränderten, insbesondere vergrößerten oder gekrümmten Elektrodenkörper (2) führt.

8. Elektrode nach Anspruch 7, **dadurch gekennzeichnet, dass** in den definierten Ausdehnungsbereichen (A) das mindestens eine Lumen (7, 23) in durch den Druck des Druckmediums entfaltbare Falten (F) gelegt ist.

9. Elektrode nach Anspruch 8, **dadurch gekennzeichnet, dass** die Falten (F) einen im Wesentlichen sternförmigen Querschnitt des Lumens (7) bilden, der durch Beaufschlagung durch das Druckmedium in einen expandierten Querschnitt übergeht.

10. Elektrode nach einem der vorgenannten Ansprüche, **dadurch gekennzeichnet, dass** das mindestens eine Lumen (7, 23) mit einem dünnwandigen, reißfesten Material ausgekleidet ist.

11. Elektrode nach einem der vorgenannten Ansprüche, **dadurch gekennzeichnet, dass** über eine Soll-Krümmungslänge des Elektrodenkörpers (2) dieser mit mindestens einem exzentrisch angeordneten, im Querschnitt sichelförmigen Lumen (23) versehen ist.

12. Elektrode nach einem der vorgenannten Ansprüche, **dadurch gekennzeichnet, dass** der Elektrodenkörper (2) zwei oder mehr verschiedene, vorzugsweise miteinander über Druckventile verbundene Lumen (7) oder Lumenabschnitte (23) aufweist, die durch unterschiedliche Druckniveaus des Druckmediums selektiv ansteuerbar sind.

13. Elektrode nach einem der vorgenannten Ansprüche, **dadurch gekennzeichnet, dass** der Elektrodenkörper (2) zwei oder mehr Lumen (7, 23) aufweist, die sich in unterschiedlich dehnbaren Materialbereichen des Elektrodenkörpers befinden.

14. Elektrode nach einem der vorgenannten Ansprüche, **dadurch gekennzeichnet, dass** durch die Beaufschlagung des Lumens (7, 23) durch das Druckmedium sich in Teilbereichen des Elektrodenkörpers (2) Funktionsteile (20), wie Häkchen, Schrauben oder Spiralen zur Verankerung des Elektrodenkörpers (2) in umgebendem Körpergewebe, ausbilden.

## Claims

1. An implantable electrode, comprising
- an elongate electrode body (2),
- at least one lumen (7, 23), which extends in the electrode body (2) over at least a portion of the length thereof, and
- a pressure device (15), by means of which the at least one lumen (7, 23) can be acted on reversibly by a pressure medium, such that the flexibility and/or shape of the electrode body (2) varies depending on the pressure of the pressure medium,
**characterised in that**
the electrode body (2) is pre-shaped, at least in portions, in a helix shape, which can be temporarily cancelled by the aforementioned application of pressure to the at least one lumen.

2. The electrode according to claim 1, **characterised in that** the at least one lumen (7, 23) is filled with a liquid pressure medium, preferably with sodium chloride solution or a gel.

3. The electrode according to claim 2, **characterised in that** the at least one lumen (7, 23) filled with the liquid pressure medium forms a closed hydraulic system together with a compressible, preferably manually actuatable reservoir (15').

4. The electrode according to claim 1 or 2, **characterised in that** an injection device (15) for the pressure medium can be detachably connected to the at least one lumen (7, 23) of the electrode.

5. The electrode according to any one of the preceding claims, **characterised in that** the at least one lumen (7, 23) in defined stiffening regions leads to an electrode body that is stiffened there in a pressure-dependent manner by the pressure of the pressure medium.

6. The electrode according to claim 5, **characterised in that** the material surrounding the lumen (7, 23) has a yield point in the stiffening regions, said yield point being adjustable preferably by a fibre reinforcement or filler reinforcement of the material.

7. The electrode according to any one of the preceding claims, **characterised in that** the at least one lumen (7, 23) in defined expansion regions (A) leads to an electrode body (2) that has a changed shape there in terms of diameter, in particular is enlarged or curved, in a pressure-dependent manner by the pressure of the pressure medium.

8. The electrode according to claim 7, **characterised in that** the at least one lumen (7, 23) is arranged in the defined expansion regions (A) in pleats (F) which can be unfolded by being acted on by the pressure medium.

9. The electrode according to claim 8, **characterised in that** the pleats (F) form a substantially star-shaped cross-section of the lumen (7) which transitions into an expanded cross-section by being acted on by the pressure medium.

10. The electrode according to any one of the preceding claims, **characterised in that** the at least one lumen (7, 23) is lined with a thin-walled, tear-resistant material.

11. The electrode according to any one of the preceding claims, **characterised in that** the electrode body (2) is provided over a target curvature length thereof with at least one eccentrically arranged lumen (23) having a sickle-shaped cross-section.

12. The electrode according to any one of the preceding claims, **characterised in that** the electrode body (2) has two or more different lumens (7) or lumen portions (23), which are preferably connected to one another via pressure valves and which can be selectively actuated by different pressure levels of the pressure medium.

13. The electrode according to any one of the preceding claims, **characterised in that** the electrode body (2) has two or more lumens (7, 23), which are disposed in material regions of the electrode body which exhibit different degrees of expansion.

14. The electrode according to any one of the preceding claims, **characterised in that** functional parts (20), such as hooks, screws or spirals, for anchoring the electrode body (2) in surrounding body tissue are formed in regions of the electrode body (2) by the aforementioned application of pressure medium to the lumen (7, 23).

## Revendications

1. Electrode implantable comprenant
- un corps d'électrode (2) étiré en longueur,
- au moins une lumière (7, 23) qui s'étend à l'intérieur sur au moins une longueur partielle du corps d'électrode (2), et
- un dispositif de mise sous pression (15), au moyen duquel l'au moins une lumière (7, 23) peut être soumise à un milieu de pression de manière réversible telle que le corps d'électrode (2) varie sa souplesse et/ou sa forme en fonction de la pression du milieu de pression,
**caractérisée en ce que**
le corps d'électrode (2) présente, au moins sur des segments, une transformation dans une forme d'hélice qui peut être annulée temporairement par la mise sous pression de l'au moins une lumière.

2. Electrode selon la revendication 1, **caractérisée en ce que** l'au moins une lumière (7, 23) est remplie avec un milieu de pression liquide, de préférence, avec une solution de chlorure de sodium ou un gel.

3. Electrode selon la revendication 2, **caractérisée en ce que** l'au moins une lumière (7, 23) remplie avec un milieu de pression liquide, forme avec un réservoir (15') compressible, de préférence pouvant être actionné manuellement, un système hydraulique fermé.

4. Electrode selon la revendication 1 ou 2, **caractérisée en ce qu'**un dispositif d'injection (15) pour le milieu de pression peut être relié de manière amovible avec l'au moins une lumière (7, 23) de l'électrode.

5. Electrode selon l'une des revendications précédentes, **caractérisée en ce que** l'au moins une lumière (7, 23) mène, dans des régions de raidissement définies par la pression du milieu de pression, vers un corps d'électrode (2) raidi à cet endroit de manière dépendante par rapport à la pression.

6. Electrode selon la revendication 5, **caractérisé en ce que**, dans les régions de raidissement, la matière entourant la lumière (7, 23) présente une limite d'élasticité qui peut être réglée par un renforcement de la matière par des fibres ou des charges.

7. Electrode selon l'une des revendications précédentes, **caractérisée en ce que** l'au moins une lumière (7, 23) mène dans des régions d'extension (A) définies par la pression du milieu de pression à un corps d'électrode (2) transformé à cet endroit dans le diamètre en fonction de la pression, notamment agrandi ou recourbé.

8. Electrode selon la revendication 7, **caractérisée en ce que**, dans des régions d'extension (A) définies, l'au moins une lumière (7, 23) est mise sous forme de plis (F) pouvant se déplier sous l'effet de la pression du milieu de pression.

9. Electrode selon la revendication 8, **caractérisée en ce que** les plis (F) constituent sensiblement une forme en étoile dans une section transversale de la lumière (7), qui se transforme en une section transversale expansée par le milieu de pression.

10. Electrode selon l'une des revendications précédentes, **caractérisée en ce que** l'au moins une lumière (7, 23) est revêtue d'une matière à parois minces résistant à la déchirure.

11. Electrode selon l'une des revendications précédentes, **caractérisée en ce que**, sur une longueur de courbure souhaitée du corps d'électrode (2), celui-ci est muni d'au moins une lumière (23) disposée de manière excentrique, en forme de faucille dans la section transversale.

12. Electrode selon l'une des revendications précédentes, **caractérisée en ce que** le corps d'électrode (2) présente deux ou plusieurs lumières (7) différentes, de préférence reliées entre elles par des soupapes de pression, ou des sections de lumière (23) qui peuvent être mises en action de manière sélective par les différents niveaux de pression du milieu de pression.

13. Electrode selon l'une des revendications précédentes, **caractérisée en ce que** le corps d'électrode (2) présente deux ou plusieurs lumières (7, 23) qui se situent dans des domaines de matière extensibles de manière différentiée du corps d'électrode.

14. Electrode selon l'une des revendications précédentes, **caractérisée en ce que**, par la soumission de la lumière (7, 23) au milieu de pression, il se forme dans des régions partielles du corps d'électrode (2), des parties fonctionnelles (20), comme des crochets, des vis ou des spirales pour l'ancrage du corps d'électrode (2) dans le tissu corporel environnant.
